# EUROPÄISCHE PATENTSCHRIFT

(11) **EP 3 049 008 B1**
(45) Veröffentlichungstag und Bekanntmachung des Hinweises auf die Patenterteilung: **26.10.2022**
(21) Anmeldenummer: 14771338.2
(22) Anmeldetag: 22.09.2014
(51) Int. Cl.: A61B 18/14, A61B 18/00

(54) **HF-CHIRURGISCHES INSTRUMENT**
HF SURGICAL INSTRUMENT
INSTRUMENT CHIRURGICAL À HAUTE FRÉQUENCE

(30) Priorität: 25.09.2013 DE 102013110595
(43) Veröffentlichungstag der Anmeldung: 03.08.2016
(73) Patentinhaber: Aesculap AG, 78532 Tuttlingen (DE)
(72) Erfinder: REICHLE, Heiko, 78532 Tuttlingen (DE); FAITSCH, Dirk, 71404 Korb (DE); HEIZMANN, Patrick, 78183 Hüfingen (DE); HAFNER, Nikolaus, 78532 Tuttlingen (DE); MASER, Thomas, 78658 Zimmern ob Rottweil (DE)
(74) Vertreter: Winter, Brandl - Partnerschaft mbB
(86) Internationale Anmeldenummer: PCT/EP2014/070132
(87) Internationale Veröffentlichungsnummer: WO 2015/044086

(56) Entgegenhaltungen:
- DE-A1- 10 205 093
- DE-A1-102011 001 372
- US-A1- 2010 292 690
- US-A1- 2011 178 515
- US-B1- 8 251 989

## Beschreibung

Die vorliegende Erfindung betrifft ein HF-chirurgisches Instrument, insbesondere ein bipolares HF-Versiegelungsinstrument, gemäß dem Oberbegriff des Anspruchs 1 und ein Verfahren zum Montieren eines solchen HF-chirurgischen Instruments gemäß dem Oberbegriff des Anspruchs 8.

Es ist bekannt, dass ein solches chirurgisches HF-Instrument im Wesentlichen aus wenigstens einer Instrumentenbranche, in der wenigstens eine Elektrode enthalten ist beziehungsweise an der wenigstens eine Elektrode angebracht ist, und aus einer elektrischen Leitung besteht, über die dem HF-Instrument und insbesondere dessen Elektrode elektrische Energie von einer externen elektrischen Energiequelle zugeführt werden kann. Bei der externen Energiequelle kann es sich beispielsweise um eine hochfrequente Wechselstromquelle in Form eines HF-Generators handeln.

Weiter ist bekannt, dass es ein solches chirurgisches HF-Instrument, insbesondere ein HF-Versiegelungsinstrument der Bipolarbauart, dazu eingesetzt werden kann, eine thermisch induzierte Veränderung oder Zerstörung von Gewebezellen herbeizuführen. Das Ziel einer solchen Gewebebeeinflussung unter Einsatz eines hochfrequenten Wechselstroms (HF) ist es häufig, eine Hämostase, Gewebedurchtrennung oder eine Gewebeversiegelung an einem menschlichen oder tierischen Körper zu bewirken. Beispielsweise können durch das HF-Versiegelungsinstrument Gefäße und Gewebestrukturen dauerhaft und sicher thermofusioniert werden. Die Thermofusion erfolgt dabei durch eine Stromapplikation über zwei sich gegenüberliegende Elektroden-bestückte Instrumentenbranchen, zwischen denen das zu behandelnde Gewebe eingespannt wird und zum anderen durch einen Anpressdruck, mit welchem das zu behandelnde Gewebe zwischen den Instrumentenbranchen zusammengedrückt beziehungsweise zusammengepresst wird.

Um die wenigstens eine Elektrode eines solchen HF-chirurgischen Instruments zuverlässig mit elektrischer Energie zu versorgen, ist es notwendig, dass die Energie zuführende elektrische Leitung und die Elektrode der Griffeinheit zuverlässig elektrisch miteinander verbunden sind. In der Praxis werden an die elektrischen Verbindungen zwischen den Elektroden und der elektrischen Leitung innerhalb eines solchen HF-chirurgischen Instruments hohe qualitative Anforderungen gestellt. Zunächst sollen die elektrischen Verbindungen für eine möglichst verlustarme Energieübertragung möglichst niederohmig ausgeführt sein. Für eine zuverlässige Funktion des HF-chirurgischen Instruments sollen die elektrischen Verbindungen innerhalb dieses Instruments zudem kontaktsicher ausgebildet sein. Weiterhin sollen die mit Gewebe in Kontakt kommenden Elektrodenseiten biokompatibel ausgebildet sein beziehungsweise auch nach der Montage des HF-Instruments biokompatibel bleiben. Des Weiteren soll ein solches HF-chirurgisches Instrument möglichst kostengünstig montierbar und bereitstellbar sein. Insbesondere bei einer bipolaren Ausführung des HF-chirurgischen Instruments soll auch die Beweglichkeit der Werkzeugelemente nicht eingeschränkt sein. Schließlich soll die elektrische Leitung zur Versorgung des HF-chirurgischen Instruments mit elektrischer Energie möglichst nur über ein Werkzeugelement erfolgen.

Häufig werden solche HF-chirurgischen Instrumente auch als Einweg-Instrumente, als so genannte single-use-Instrumente, gefertigt und eingesetzt. Insbesondere bei einer solchen Ausführungsvariante eines HF-chirurgischen Instruments kann es zudem wünschenswert sein, den Montageaufwand zur Bereitstellung des Instruments möglichst gering zu halten, um vergleichsweise geringe Bereitstellungskosten zu erreichen.

Aus dem Stand der Technik sind HF-chirurgische Instrumente bekannt, bei denen die elektrischen Verbindungen durch Lötverbindungen, dass heißt durch eine stoffschlüssige Verbindung, gefertigt beziehungsweise hergestellt werden. Diese Art von elektrischer Verbindung zwischen der elektrischen Leitung und der Elektrode erfüllt zwar die oben genannten qualitativen Anforderungen hinsichtlich der Zuverlässigkeit, den Übertragungsverlusten und weitestgehend der Kontaktsicherheit. Allerdings erweisen sich Lötverbindungen in diesem Zusammenhang insbesondere dann als nachteilig, wenn die Elektrode beziehungsweise die Elektroden in segmentierter Anordnung beziehungsweise segmental in oder an der Handgriffeinheit des HF-chirurgischen Instruments vorliegen, die wiederum einzeln bestromt werden müssen. Denn meist ist die elektrische Leitung als mehradrige Leitung ausgebildet, so dass mehrere Adern dieser Leitung mit jedem einzelnen Segment der segmentalen Elektrode verlötet werden müssen. Das heißt, je mehr einzelne Elektrodensegmente in beziehungsweise an der Griffeinheit vorgesehen sind, desto häufiger muss der Lötprozess zum Verbinden der elektrischen Leitung mit den einzelnen Elektroden wiederholt werden. Mit einer ansteigenden Anzahl einzelner Elektrodensegmente wird es bei beschränkten Platzverhältnissen innerhalb des HF-chirurgischen Instruments aber auch zunehmend schwieriger, die einzelnen Adern der elektrischen Leitung durch das HF-chirurgische Instrument hin zu den einzelnen Elektrodenkörpern zu führen. Deshalb erfordern diese Lötverbindungen einen gewissen Zeitaufwand bei der Fertigung beziehungsweise Montage eines HF-chirurgischen Instruments, wodurch die Herstellungskosten eines derart gefertigten HF-chirurgischen Instruments vergleichsweise hoch sind.
EP2403422B1 offenbart ein chirurgisches Instrument zum Trennen und Koagulieren von Gewebe. Das Instrument weist einen Griff und einen Werkzeugeinsatz auf. Der Werkzeugeinsatz hat zwei Branchen zum Greifen von Gewebe, wobei mindestens eine der Branchen zwischen einer offenen und einer geschlossenen Position bewegbar ist.
US8251989 offenbart ein monopolares elektrochirurgisches HF- Instrument mit einem Griffgehäuse. Weiter offenbart das Dokument ein elektrisches Schaltsystem welches automatisch erkennt, was für Bestromungsbedingungen notwendig sind
US20110178515 offenbart ein elektrochirurgisches Instrument zur Gewebebehandlung in Anwesenheit einer Flüssigkeit.

Des Weiteren soll ein HF-chirurgisches Instrument häufig in einem Reinraum gefertigt beziehungsweise montiert werden. In einem solchen Reinraum ist das Löten jedoch nur bedingt möglich, meist unter Einhaltung strenger Anforderungen hinsichtlich

Arbeits- und/oder Prozesssicherheit. Auch die Einhaltung dieser strengen Anforderungen ist mit einem finanziellen Aufwand verbunden, wodurch die Herstellungskosten des Instruments vergleichsweise hoch sind.

Darüber hinaus können die eingesetzten Elektroden eine vergleichsweise hohe Wärmekapazität aufweisen, so dass ein Aufschmelzen des Materials und somit die Herstellung der Lötverbindung nur vergleichsweise zeitaufwendig bewerkstelligt werden kann.

Der Einsatz von Lötverbindungen als elektrische Verbindung zwischen der elektrischen Leitung und der wenigstens einen Elektrode der Griffeinheit kann sich aber auch dann als nachteilig erweisen, wenn die Elektrode aus einem Material gefertigt werden soll, das sich nur bedingt, nicht direkt oder gar nicht für das Löten eignet. Somit unterwirft der Einsatz von Lötverbindungen die Bereitstellung und Montage von HF-chirurgischen Instrumenten einigen Einschränkungen.

Dem gegenüber ist es die Aufgabe der Erfindung, den Montageaufwand für ein HF-chirurgisches Instrument unter Einsatz konstruktiv möglichst einfacher Mittel zu reduzieren. Ein Ziel ist es hierbei, die Montage- beziehungsweise Bereitstellungskosten eines solchen HF-chirurgischen Instruments durch eine Vereinfachung von dessen Montage zu reduzieren. Ein weiteres/anderes Ziel ist es, eine vergleichsweise kostengünstig bereitstellbare elektrische Verbindung einzelner Komponenten des HF-chirurgischen Instruments zu realisieren.

Diese Aufgabe wird gelöst durch ein HF-chirurgisches Instrument mit den Merkmalen des Anspruchs 1 beziehungsweise durch ein Verfahren zum Montieren eines HF-chirurgischen Instruments mit den Merkmalen des Anspruchs 8.

Vorteilhafte Weiterbildungen der Erfindung sind in den Unteransprüchen angegeben.

Gemäß eines ersten unabhängigen Aspekts der Erfindung weist ein HF-chirurgisches Instrument zwei zueinander in eine Schließstellung bewegbare Instrumentenbranchen auf, die jeweils mit zumindest einer Elektrode bestückt sind. Die Elektroden sind mittels einer Energiequelle mit elektrischer Energie versorgbar beziehungsweise können von einer Energiequelle mit elektrischer Energie versorgt werden. Erfindungsgemäß weist das HF-chirurgische Instrument eine separat ausgebildete Überbrückungseinrichtung auf, bei der es sich insbesondere um eine, vorzugsweise maschinell bestückbare, Leiterplatte handeln kann. Die Überbrückungseinrichtung ist über zumindest einen Anschluss oder zumindest eine Leitung mit der Energiequelle verbindbar und weist zur Versorgung der zumindest einen Elektrode mit elektrischer Energie zumindest eine elektrische leitfähige Kontaktstelle auf. Die separat zu dem HF-chirurgischen Instrument und/oder zu der Elektrode separat ausgebildete Überbrückungseinrichtung ist in die zumindest eine der beiden Instrumentenbranchen derart leinbringar, dass die zumindest eine elektrisch leitfähige Kontaktstelle die zumindest eine Elektrode elektrisch kontaktiert bzw. mit dieser in Anlage bringbar ist und wenn die Überbrückungseinrichtung über eine axiale Öffnung an der proximalen Seite der Instrumentenbranche in axialer Richtung in die Überbrückungseinrichtung einschiebbar ist.

In anderen Worten weist das erfindungsgemäße HF-chirurgische Instrument eine separat zu der Elektrode ausgebildete Überbrückungseinrichtung auf, mittels der eine an das HF-chirurgische Instrument heran geführte beziehungsweise in dieses mündende elektrische Leitung mit einer elektrischen Kontaktstelle einer Elektrode elektrisch leitend verbunden werden kann. Wenigstens bei der elektrischen Verbindung zwischen der Überbrückungseinrichtung und der Kontaktstelle der Elektrode kann es sich um eine stoffunschlüssig ausgebildete Verbindung handeln. Unter stoffunschlüssiger elektrischer Verbindung ist in diesem Zusammenhang jede Art von elektrisch leitfähiger Verbindung zu verstehen, bei der die jeweiligen Verbindungspartner nicht durch zwischen diesen wirkenden atomare oder molekulare Kräfte zusammengehalten werden. Das heißt, dass die stoffunschlüssig ausgebildete beziehungsweise hergestellte Überbrückungseinrichtung wenigstens zwischen sich selbst und der Kontaktstelle der wenigstens einen Elektrode der Griffeinheit keine Löt-, Schweiß- oder Klebeverbindung oder dergleichen Verbindungsart aufweist. Insbesondere handelt es sich bei dieser erfindungsgemäß stoffunschlüssig ausgebildeten Verbindung, im Gegensatz zu einer Lötverbindung, um eine zerstörungsfrei lösbare Verbindung. Die stoffunschlüssige Verbindung zwischen der Überbrückungseinrichtung und der Vielzahl von segmentierten bietet den Vorteil, dass eine Vielzahl von einzelnen Lötverbindungen zwischen der elektrischen Leitung und der Vielzahl von segmentierten Elektroden eingespart werden kann. Gegenüber der im Stand der Technik vorgeschlagenen Lösung, bei der an jeder aus der Vielzahl von Elektroden ein Blech angeordnet wird, kann hier der Montageaufwand signifikant reduziert werden.

Unter Überbrückungseinrichtung ist in diesem Zusammenhang eine wenigstens abschnittsweise elektrisch leitfähige Einrichtung, Adapter oder Komponente zu verstehen, mit der eine räumliche Distanz zwischen der zu dem HF-chirurgischen

Instrument geführten beziehungsweise in ein Gehäuse desselben hinein geführten elektrischen Leitung und der wenigstens einen mit elektrischer Energie zu versorgenden Elektrode überwunden beziehungsweise überbrückt werden kann. Das heißt, dass die erfindungsgemäße Überbrückungseinrichtung dazu konfiguriert ist, eine räumliche Verbindung zwischen der elektrischen Leitung und der wenigstens einen Elektrode des HF-chirurgischen Instruments zu bewerkstelligen beziehungsweise herzustellen.

Die zu dem chirurgischen Instrument geführte beziehungsweise die in ein Gehäuse des chirurgischen Instrument oder der Griffeinheit mündende elektrische Leitung kann dazu eingerichtet sein, elektrische Energie von einer externen Energiequelle, vorzugsweise von einem HF-Generator, zu dem HF-chirurgischen Instrument beziehungsweise zu der wenigstens einen Elektrode elektrisch zu leiten. Dabei kann die elektrische Leitung mehradrig ausgebildet sein und zum elektrischen Anschluss an den HF-Generator und über diesen an das jeweilige Stromnetz der jeweiligen Einsatzregion angepasst sein.

Die separat beziehungsweise als separates Bauteil zu der wenigstens einen Elektrode und/oder zu der Griffeinheit ausgebildete und (separat) in das HF-chirurgische Instrument einbringbare Überbrückungseinrichtung ermöglicht es vorteilhafterweise, dass die Überbrückungseinrichtung separat zu den übrigen Komponenten des HF-chirurgischen Instruments gefertigt beziehungsweise auch vorgefertigt oder vormontiert werden kann. Somit kann die Überbrückungseinrichtung zur Endmontage des HF-chirurgischen Instruments in dieses eingebracht beziehungsweise eingesetzt oder eingeschoben werden und gegebenenfalls daran befestigt werden.

Das heißt, dass keine Lötverbindung zwischen der wenigstens einen Elektrode und der elektrischen Leitung hergestellt werden muss, was in einem Reinraum unter Umständen, beispielsweise aus Gründen der Arbeitssicherheit, problematisch wäre. Zudem kann durch den Verzicht auf eine manuell herstellbare Lötverbindung der Arbeits- und Zeitaufwand signifikant reduziert werden, wodurch auch die Herstellungskosten für ein HF-chirurgisches Instrument reduziert werden können.

Darüber hinaus kann durch den Einsatz einer maschinell bestückten Leiterplatte eine vergleichsweise hohe Reproduzierbarkeit bei der Fertigung der Leitplatte und somit bei der Montage des HF-chirurgischen Instruments erzielt werden. Insbesondere gegenüber einer manuell gefertigten Lötverbindung kann damit eine höhere Produktsicherheit und Produktqualität erreicht werden, wobei in Abhängigkeit der zu fertigenden Stückzahlen zusätzlich die Fertigungskosten gesenkt werden können.

Durch die kabellose Verbindung zwischen der elektrischen Leitung und der mit elektrischer Energie zu versorgenden Elektrode wird die Montage erleichtert und bei einer Vielzahl an Elektroden überhaupt erst ermöglicht. Ferner kann auch die Handhabung beziehungsweise die Beweglichkeit der Instrumentenbranche verbessert werden, da keine oder zumindest vergleichsweise wenige elektrischen Leitungen (Kabel) durch die Instrumentenbranche geführt werden müssen. Beispielsweise kann das HF-chirurgische Instrument auch eine Vielzahl von segmentierten beziehungsweise segmentaler Elektroden aufweisen, die alle durch die Überbrückungseinrichtung stoffunschlüssig mit der elektrischen Leitung verbindbar sind. Das heißt, dass das Griffteil des HF-chirurgischen Instruments auch mehrere segmental angeordnete Elektroden aufweisen kann, die mittels der Überbrückungseinrichtung auf stoffunschlüssige Weise mit der elektrischen Leitung verbindbar sind. Die stoffunschlüssige Verbindung zwischen der Überbrückungseinrichtung und der Vielzahl von segmentierten Elektroden bietet den Vorteil, dass eine Vielzahl von einzelnen Lötverbindungen zwischen der elektrischen Leitung und der Vielzahl von segmentierten Elektroden eingespart werden kann. Gegenüber der im Stand der Technik vorgeschlagenen Lösung, bei der an jeder aus der Vielzahl von Elektroden ein Blech angeordnet wird, kann hier der Montageaufwand signifikant reduziert werden.

Zudem bewirkt die Erfindung vorteilhafterweise, dass eine vergleichsweise geringe Fehlerquote bei der elektrischen Paarung der elektrischen Leitung und der Elektrode auftritt. Darüber hinaus wird durch die von dem HF-chirurgischen Gerät lösbare Verbindung auch die Wartung desselben verbessert, da die Überbrückungsvorrichtung ohne nennenswerten Aufwand ausgetauscht werden kann. Des Weiteren können auch räumlich schwer erreichbare Elektroden auf einfache Weise mit elektrischer Energie versorgt werden.

Da sich mittels der zwischengeschalteten Leiterplatte bzw. Überbrückungseinrichtung zur Elektrodenkontaktierung der Automatisierungsgrad gravierend steigern lässt, kann die Produktion wirtschaftlicher erfolgen, was insbesondere bei Single-Use-Instrumenten von großer Bedeutung ist.

Es hat sich als besonders zweckmäßig erwiesen, wenn die zumindest eine elektrisch leitfähige Kontaktstelle der Überbrückungseinrichtung und/oder die Elektrode ein elektrisch leitendes Federkontaktelement aufweist, das einen elektrisch leitfähigen Druckkontakt zwischen der Kontaktstelle und der Elektrode bewirkt. Auf diese Weise können fertigungsbedingte Toleranzen ausgeglichen werden, die beispielsweise an einem Gehäuse des HF-chirurgischen, an der Überbrückungseinrichtung oder der Elektrode auftreten können. Zudem ermöglicht ein solcher Druckkontakt eine besonders zuverlässige elektrisch leitende Verbindung zwischen der Elektrode und einer elektrischen Energiequelle. Ein solcher Druckkontakt ermöglicht eine besonders einfache Montage des HF-chirurgischen Instruments.

Ein anderer, gegebenenfalls unabhängiger oder zusätzlicher Aspekt der Erfindung sieht vor, dass die Überbrückungseinrichtung, wenn diese lose in die Instrumentenbranche eingebracht wird, über den Druckkontakt zwischen dem zumindest einen Federkontakt und der zumindest einen Elektrode kraftschlüssig in der Instrumentenbranche fixiert wird. Dabei kann der Druckkontakt beispielsweise so ausgebildet sein, dass die Überbrückungseinrichtung in dem HF-chirurgischen Instrument, insbesondere zwischen der Elektrode und einem Gehäuseabschnitt des HF-chirurgischen Instruments, festgeklemmt werden kann. Mit einer solchen Konfiguration der Überbrückungseinrichtung beziehungsweise des Druckkontakts verfügt das Federkontaktelement der Überbrückungseinrichtung über eine Doppelfunktion, die eine elektrisch leitende Verbindung und gleichzeitig eine kraftschlüssige Fixierung der Überbrückungseinrichtung in dem HF-chirurgischen Instrument, beispielsweise innerhalb eines Gehäuseabschnitts desselben, ermöglicht. Durch diese kraftschlüssige Fixierung ist es zudem möglich, die Überbrückungseinrichtung ohne den Einsatz eines zusätzlichen Werkzeugs zu montieren. Auch eine zerstörungsfreie Demontage der Überbrückungseinrichtung ohne den Einsatz eines Werkzeugs wird dadurch ermöglicht.

Gemäß einer vorteilhaften Weiterbildung der Erfindung kann das zumindest eine Federkontaktelement in einer vorgesehenen Kontaktposition einen lösbaren Rasteingriff zwischen Instrumentenbranche und Überbrückungseinrichtung herstellen. Beispielsweise kann hierfür eine Vertiefung in einem Gehäuseabschnitt des HF-chirurgischen Instruments ausgebildet sein, in den die Überbrückungseinrichtung eingelegt werden kann. Auf diese Weise kann, ohne den Einsatz eines Werkzeugs, eine kraft- und formschlüssige Fixierung der Überbrückungseinrichtung in dem HF-chirurgischen Instrument erreicht werden.

Es hat sich als besonders vorteilhaft erwiesen, wenn die Überbrückungseinrichtung über eine axiale Öffnung an der proximalen Seite der Instrumentenbranche in axialer Richtung in die Überbrückungseinrichtung einschiebbar ist. Dadurch ist ein besonders einfacher Aufbau der Instrumentenbranche möglich.

Gemäß einem Aspekt der Erfindung kann die Überbrückungseinrichtung von der Instrumentenbrancheaußenseite, z.B. von der Unterseite der unteren Instrumentenbranche, in die Instrumentenbranche bzw. den Elektrodenhalter derart eingesetzt werden, dass die zumindest eine Kontaktstelle in Anlage mit der zumindest einen Elektrode gelangt und diese elektrisch kontaktiert. Die Fixierung der Überbrückungseinrichtung an der Instrumentenbranche bzw. dem Elektrodenhalter kann auf unterschiedliche Weise, zum Beispiel formschlüssig, mittels Stifte oder Schrauben, erfolgen.

Ein anderer, gegebenenfalls unabhängiger oder zusätzlicher Aspekt der Erfindung sieht vor, dass der zumindest eine elektrisch leitfähige Anschluss der Überbrückungseinrichtung ein Klemm-Schneid-Element zur schneidenden und/oder klemmenden elektrischen Verbindung mit einer mit der elektrischen Energiequelle verbundenen elektrischen Leitung aufweist. Diese Verbindung kann alternativ auch durch Crimpen bewerkstelligt werden. Dadurch kann auch bei der elektrisch leitfähigen Verbindung zwischen der elektrischen Energiequelle beziehungsweise der elektrischen Leitung und der Überbrückungseinrichtung auf eine (stoffschlüssige) Lötverbindung verzichtet werden. Dadurch können die Litzen der elektrischen Leitung und die Klemm-Schneid-Elemente oder -Kontakte auch im Reinraum miteinander verbunden werden. In diesem Fall liegt weder an der Schnittstelle zur elektrischen Zuführleitung noch an der Schnittstelle zu der zumindest einen Elektrode eine stoffschlüssige Verbindung vor, was die Montage vereinfacht bzw. überhaupt erst möglich macht. Anders ausgedrückt sind alle Kontakte und Anschlüsse lötfrei.

Alternativ dazu, kann der zumindest eine elektrisch leitfähige Anschluss durch eine Lötöse zur stoffschlüssigen, elektrischen Verbindung mit einer mit der Energiequelle verbundenen elektrischen Leitung ausgebildet sein. Dadurch kann ein vergleichsweise sicherer elektrisch leitfähiger Kontakt zwischen der elektrischen Energiequelle und der Überbrückungseinrichtung sichergestellt werden. Zudem ermöglicht die Lötöse eine vergleichsweise einfach zu fertigende Lötverbindung an einer relativ leicht zugänglichen Stelle der Überbrückungseinrichtung. Vorteilhafterweise kann die Lötöse so an der Überbrückungseinrichtung angeordnet sein, dass eine kabellose elektrische Kontaktierung im gesamten Endbereich des HF-chirurgischen Instruments möglich ist.

Ein anderer gegebenenfalls unabhängiger oder zusätzlicher Aspekt der Erfindung sieht vor, dass die eine der beiden Instrumentenbranchen mit der Energiequelle über eine Leitung direkt verbunden ist und die andere der beiden Instrumentenbranchen mittelbar über die eine Instrumentenbranche mit elektrischer Energie versorgt wird. Dabei kann an der Überbrückungseinrichtung der einen Instrumentenbranche ein erster Kontakt, vorzugsweise ein Federkontakt, und an der Überbrückungseinrichtung der anderen Instrumentenbranche ein zweiter Kontakt, vorzugsweise Federkontakt, vorgesehen sein, die zumindest in der Schließstellung der beiden Instrumentenbranchen einen elektrischen leitfähigen Kontakt eingehen. Dadurch ist es vorteilhafterweise möglich, auf eine separate Energiezuführung für die andere Instrumentenbranche zu verzichten, wodurch eine einfache und synchrone Bestromung der Elektroden beider Instrumentenbranchen möglich ist. Dies hat sich auch aus sicherheitstechnischen Gründen als vorteilhaft erwiesen, da die sich gegenüberliegenden Elektroden nur in der Schließstellung, das heißt wenn die zusammenwirkenden Kontakte durch einen gegenseitigen Kontakt betätigt werden, bestromt werden. Darüber hinaus wird die Beweglichkeit der beiden Instrumentenbranchen, insbesondere die Beweglichkeit der mittelbar über die eine Instrumentenbranche mit elektrischer Energie versorgten Instrumentenbranche, enorm verbesserst, da diese überhaupt nicht über eine (eigene) elektrische Leitung angesteuert wird.

Die Erfindung bezieht sich auch auf ein Verfahren mit den merkmalen des Anspruchs 8 zum Montieren eines HF-chirurgischen Instruments, das zwei zueinander in eine Schließstellung bewegbare Instrumentenbranchen aufweist, die jeweils mit zumindest einer Elektrode bestückbar sind und mit elektrischer Energie von einer Energiequelle versorgt werden können.

Durch den Einsatz dieses (Montage-)Verfahrens kann ein HF-chirurgisches Instrument montiert beziehungsweise bereitgestellt werden, das sich durch eine besonders vorteilhaft einfache Herstellbarkeit und Handhabung auszeichnet.

Die Erfindung wird nachstehend anhand eines bevorzugten Ausführungsbeispiels unter Bezugnahme auf die begleitenden Figuren näher erläutert. Es zeigen:
Figur 1 eine perspektivische Seitenansicht eines erfindungsgemäßen HF-chirurgischen Instruments, das an einer externen elektrischen Energiequelle angeschlossen ist,
Figur 2 eine Schnittansicht durch ein erfindungsgemäßes HF-chirurgisches Instrument, insbesondere einen Schnitt durch eine Griffeinheit beziehungsweise Instrumentenbranche des HF-chirurgischen Instruments,
Figur 3 eine Draufsicht auf eine teilweise frei geschnittene Überbrückungseinrichtung eines erfindungsgemäßen HF-chirurgischen Instruments,
Figur 4 eine Draufsicht auf eine teilweise frei geschnittene Überbrückungseinrichtung eines erfindungsgemäßen HF-chirurgischen Instruments, bei der zu Illustrationszwecken einzelne Bauelemente nicht dargestellt sind, und
Figur 5 eine perspektivische Seitenansicht einer Instrumentenbranche mit segmentierten Elektroden eines erfindungsgemäßen HF-chirurgischen Instruments.

Gleiche oder ähnliche Komponenten sind durchgängig mit gleichen Bezugszeichen versehen.

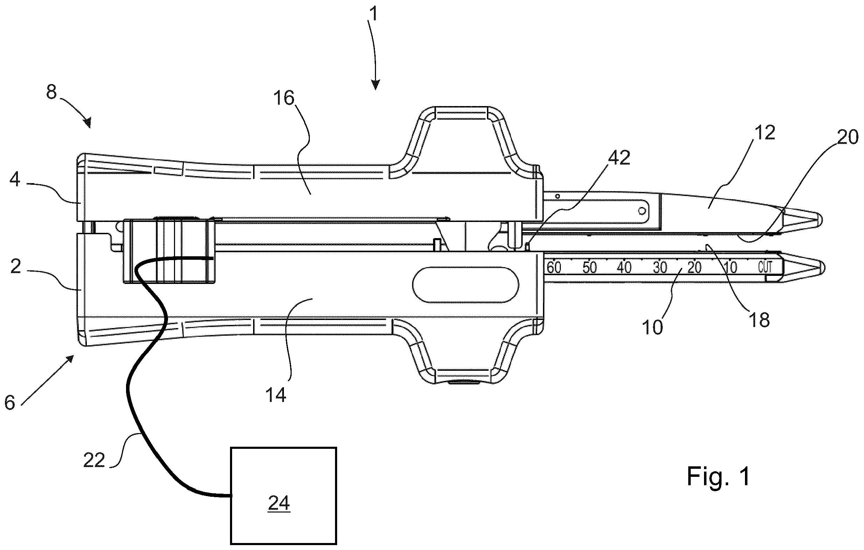

Figur 1 zeigt ein Ausführungsbeispiel eines erfindungsgemäßen HF-chirurgischen Instruments 1 in Form eines HF-Versiegelungsinstruments der bipolaren Bauart in einer perspektivischen Seitenansicht. Dieses HF-chirurgische Instrument 1 ermöglicht es einem Anwender, eine thermisch induzierte Veränderung oder Zerstörung von Gewebezellen herbeizuführen. Beispielsweise können mit dem HF-chirurgischen Instrument 1 Gefäße und Gewebestrukturen eines menschlichen Körpers dauerhaft und sicher zu thermofusioniert werden.

Das in Figur 1 dargestellte HF-chirurgische Instrument 1 weist zwei trennbar miteinander scharnierartig verbundene (verbindbare) Instrumentenhälften 2, 4 auf, die jeweils aus einer unteren Griffeinheit 6, 8 mit jeweils einer in Instrumentenlängsrichtung (aus Anwendersicht) distal hierzu angeordneten Instrumentenbranche 10, 12 bestehen. In Figur 1 ist zu erkennen, dass sich die Instrumentenbranchen 10 und 12 gegenüberliegen, um dazwischen das zu behandelnde Gewebe (nicht gezeigt) einzuspannen. Die in Figur 1 untere Griffeinheit 6 und die in Figur 1 obere Griffeinheit 8 weisen jeweils eine für einen Anwender ergonomisch geformte Griffschale 14, 16 auf, die beispielsweise aus einem für den medizinischen Einsatzbereich geeigneten Kunststoff- oder Blechmaterial gefertigt sind.

Die untere Instrumentenbranche 10 weist an einer der anderen, oberen Instrumentenbranche 12 zugewandten Seite eine mit elektrischer Energie betreibbare Elektrode 18 auf, die in Figur 1 nur andeutungsweise erkennbar ist. Die obere Instrumentenbranche 12 weist ebenfalls an einer der anderen, unteren Instrumentenbranche 10 zugewandten Seite eine weitere Elektrode 20 auf, die in Figur 1 ebenfalls nur andeutungsweise erkennbar ist. Zur Versorgung des HF-chirurgischen Instruments 1 mit elektrischer Energie weist dieses beziehungsweise weist eine der Griffeinheiten 6 oder 8 eine nicht weiter bezeichnete Gehäuseöffnung auf, durch die eine elektrische Leitung 22 zu dem beziehungsweise in das HF-chirurgische Instrument 1 hinein geführt ist. Die elektrische Leitung 22 ist mit einer externen elektrischen Energiequelle 24 elektrisch verbunden, bei der es sich in diesem Ausführungsbeispiel um einen HF-Generator zur Erzeugung von hochfrequentem Wechselstrom (HF-Strom) handelt. Die Versorgung der beiden Elektroden 18, 20 durch die elektrische Energiequelle 24 mit elektrischer Energie wird weiter unten detailliert beschrieben.

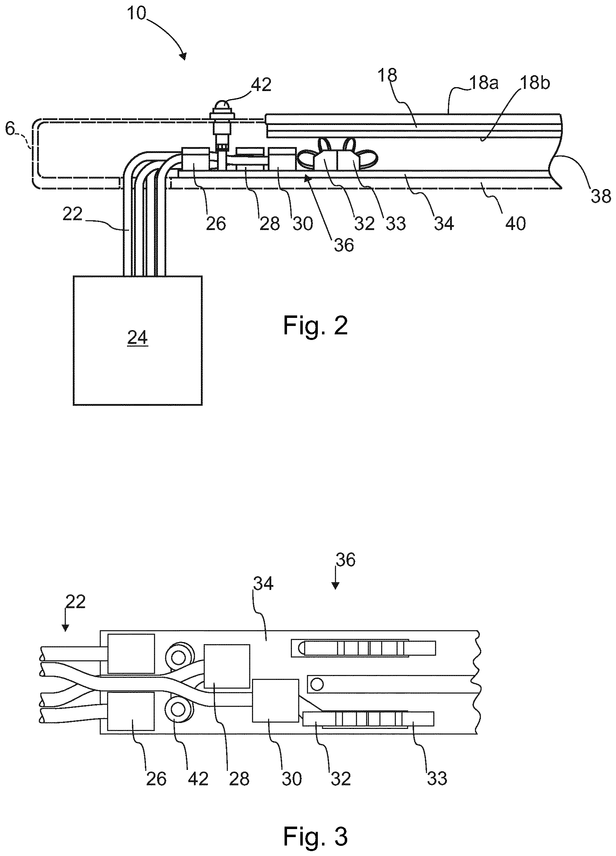

Figur 2 zeigt eine Schnittansicht durch das HF-chirurgische Instrument 1, insbesondere einen Schnitt durch die untere Griffeinheit 6 beziehungsweise die untere Instrumentenbranche 10 des HF-chirurgischen Instruments 1. Es ist zu erkennen, dass die Elektrode 18 in die Instrumentenbranche 10 eingelagert ist, und eine nach außen freigelegte Wirkfläche 18a aufweist, die für einen Kontakt mit dem zu behandelnden Gewebe eingerichtet ist. Weiter ist zu erkennen, dass die elektrische Leitung 22 von der elektrischen Energiequelle 24 in das HF-chirurgische Instrument 1 geführt ist und in drei Kontaktelementen 26, 28, 30 mündet, bei denen es sich in diesem Ausführungsbeispiel um ein Schneid- und/oder Klemmelement zur schneidenden und/oder klemmenden, elektrisch leitfähigen Befestigung der Leitung 22 handelt. Durch diese Art von Kontaktelementen 26, 28, 30 ist es jeweils möglich, die elektrische Leitung 22 gegebenenfalls ohne ein vorheriges Entfernen seiner den elektrischen Leiter umgebenden Isolierung direkt in das jeweilige Kontaktelement 26, 28 oder 30 einzulegen und daran zu befestigten. Denn die drei Kontaktelemente 26, 28, 30 weisen jeweils ein Schneideteil auf, das sich gegebenenfalls durch die Isolierung der elektrischen Leitung 22 hindurch schneidet und auf diese Weise eine elektrische Verbindung zwischen sich und den einzelnen, einen elektrischen Strom leitenden Adern der elektrischen Leitung 22 herstellt. Darüber hinaus weisen die Kontaktelemente 26, 28, 30 ein Klemmteil 26a, 28a, 30a auf, durch dessen klemmenden Eingriff die elektrische Leitung an den Kontaktelementen 26, 28, 30 klemmend befestigt wird.

Weiter geht aus Figur 2 hervor, dass die als Schneid- und/oder Klemmelement ausgeführten Kontaktelemente 26, 28, 30 in sich berührenden Kontakt, das heißt in Anlage mit Federkontaktelementen 32, 33 befinden, die in Figur distal neben dem Kontaktelement 30 angeordnet sind. Insbesondere besteht zwischen dem Kontaktelement 30, an dem die elektrische Leitung 22 befestigt ist, und dem Federkontaktelement 32 durch eine direkte Berührung dieser beiden Bauelemente eine elektrische Verbindung. Auf diese Weise kann die von der elektrischen Energiequelle 24 erzeugte elektrische Energie über die elektrische Leitung 22 und das Kontaktelement 30 zu dem Federkontaktelement 32 geleitet werden. In Figur 2 ist weiter zu erkennen, dass ein Federteil 32a des Federkontaktelements 32 derart innerhalb der Griffeinheit 6 angeordnet ist, dass es von unten gegen eine untere Oberflächenseite 18b die Elektrode 18 gedrückt wird. Auf diese Weise befindet sich das Federkontaktelement 32 mit der der Wirkfläche 18a gegenüberliegenden Seite 18b der Elektrode 18 in elektrisch leitender Anlage, um so die von der elektrischen Energiequelle 24 erzeugte elektrische Energie über die elektrische Leitung 22, dem Kontaktelement 30 und dem Federkontaktelement 32 zur Elektrode 18 zuzuleiten.

Figur 2 zeigt auch, dass die für die Übertragung der elektrischen Energie Elemente in diesem Ausführungsbeispiel verwendeten Elemente, nämlich die Kontaktelemente 26, 28, 30 und das Federkontaktelement 32, auf einer gemeinsamen Seite einer Leiterplatte 34 angeordnet sind. Die Leiterplatte 34 und die darauf angebrachten beziehungsweise befestigten Elemente 26, 28, 30 und 32 bilden auf diese Weise gemeinsam eine Überbrückungseinrichtung 36, die die räumliche Distanz zwischen der in das HF-chirurgische Instrument 1 mündenden elektrischen Leitung 22 und der zu bestromenden Elektrode 18 überbrückt beziehungsweise überwindet, um auf diese Weise eine elektrische Verbindung zwischen diesen beiden Bauteilen herzustellen.

Bei der Leiterplatte 34 handelt es sich vorzugsweise um ein vorgefertigtes, ebenfalls vorzugsweise maschinell gefertigtes Bauelement, das bei der Endmontage des HF-chirurgischen Instruments 1 in eine proximal angeordnete Aufnahmeöffnung 38 eingebracht oder eingelegt beziehungsweise eingeschoben wird, die zwischen der Elektrode 18 und einer zur Griffeinheit 6 gehörenden Gehäusewand 40 gebildet ist. Dabei wird die zwischen der Elektrode 18 und der Gehäusewand 40 angeordnete Leiterplatte 34 durch die sich an der (fest montierten) Elektrode 18 abstützenden Federkontaktelemente 32, 33 in Richtung der Gehäusewand 40 gespannt, wodurch die Leiterplatte 34 innerhalb der Aufnahmeöffnung kraftschlüssig fixiert wird, so dass keine weitere Befestigung der Leitplatte 34 vorgesehen werden muss. Optional kann jedoch eine weitere (nicht dargestellte) Befestigungsmöglichkeit vorgesehen sein.

Weiter ist in Figur 2 ein federnd beziehungsweise schwimmend gelagertes Kontaktelement 42 gezeigt, das ebenfalls mit der elektrischen Leitung 22 elektrisch verbunden ist. Das federnd gelagerte Kontaktelement 42 dient dazu, eine elektrische Verbindung zu der anderen, in Figur 2 nicht dargestellten Griffeinheit 8 oder Instrumentenbranche 12 beziehungsweise zu der daran angeordneten Elektrode 20 durch eine kontaktierende Anlage an dieser herzustellen. Auf diese Weise kann bei einer Annäherung der beiden Griffeinheiten 6, 8 beziehungsweise der Instrumentenbranchen 10, 12 über die Leiterplatte 34 beziehungsweise durch das darauf angeordnete federnd gelagerte Kontaktelement 42 eine elektrische Verbindung zwischen der elektrischen Leitung 22 und der Elektrode 20 hergestellt werden. Das federnd gelagerte Kontaktelement 42 ist beispielsweise in Figur 1 gezeigt, die eine perspektivische Seitenansicht des HF-chirurgischen Instruments 1 zeigt. Der Federkontakt 42 hat einen federvorgespannten Kontaktstift oder eine Federzunge, der/die sich von einer der Instrumentenhälften 18 in Richtung hin zur anderen Instrumentenhälfte 18 erstreckt, um von dieser betätigt werden zu können. Erst beim Schließen der Instrumentenbranchen 10, 12 kommt der Federkontakt 42 mit einem entsprechenden Kontaktelement an der anderen Instrumentenbranche 12 in Kontakt. Der Kontakt auf der anderen Instrumentenbranche ist elektrisch mit der Elektrode 20 bzw. mit deren Elektrodensegmente in Verbindung. Somit wird erst mit oder kurz vor Erreichen der vorbestimmten Schließkraft ein elektrischer Strom von einer Energiequelle, vorzugsweise einem externen HF-Generator an die beiden Instrumentenbranchen 10, 12 freigegeben. Auf diese Weise wird die Elektrode 20 der anderen (oberen) Instrumentenbranchen 12 mittelbar über die eine (untere) Instrumentenbranche 10, genauer gesagt, über die darin vorgesehene Überbrückungseinrichtung 36 angesteuert werden.

Figur 3 zeigt eine Draufsicht auf die teilweise frei geschnittene Überbrückungseinrichtung 36 in Form der Leiterplatte 34 des HF-chirurgischen Instruments 1. Es ist zu erkennen, dass die Leiterplatte 34 derart mit den Kontaktelementen 26, 28, 30 und dem federnd gelagerten Kontaktelement 42 bestückt ist, dass die elektrische Leitung 22 mit jedem dieser Elemente auf einfache Weise verbundbar ist bzw. verbunden werden kann.

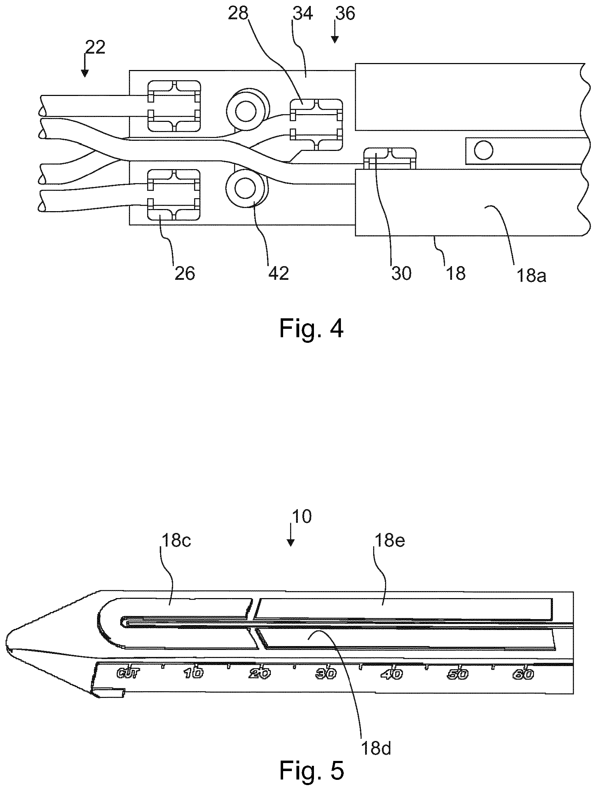

Aus Figur 4, die eine Draufsicht auf die teilweise frei geschnittene Überbrückungseinrichtung in Form der Leiterplatte 34 des HF-chirurgischen Instruments 1 zeigt, bei der zu Illustrationszwecken einzelne Bauelemente nicht dargestellt sind, geht die Funktionsweise der jeweils als Schneid- und/oder Klemmelement ausgeführten Kontaktelemente 26, 28, 30 hervor. Insbesondere ist in Figur 4 jeweils eine obere Abdeckung (nicht näher bezeichnet) der als Schneid- und/oder Klemmelemente ausgeführten Kontaktelemente 26, 28, 30 nicht dargestellt, um das Einlegen und Festklemmen von einzelnen Adern (nicht näher bezeichnet) der elektrischen Leitung 22 mittels des Klemmteils 26a, 28a, 30a besser darstellen zu können. Weiter ist in Figur 4 zu erkennen, dass die federnd gelagerte Kontaktelemente 42 in elektrischer Verbindung mit dem Kontaktelement 30 steht, um die Elektrode 20 der Instrumentenbranche 10 mit elektrischer Energie versorgen zu können.

In Figur 5, die eine perspektivische Seitenansicht der Instrumentenbranche 10 mit segmentierten beziehungsweise segmentalen Elektroden 18c, 18d und 18e des HF-chirurgischen Instruments 1 zeigt, ist zu erkennen, dass auch mehrere vereinzelt angeordnete, das heißt segmentierte Elektroden mittels der Überbrückungseinrichtung in Form der Leiterplatte 34 mit elektrischer Energie versorgt werden können. Hierfür sind lediglich mehrere elektrische leitfähige Kontaktelemente beziehungsweise Federkontaktelemente zur Kontaktierung der einzelnen Segmente der Elektrode notwendig. Um eine ausreichende Kontaktierung sicherzustellen, können anstelle von einer auch zwei oder mehrere Federkontaktelemente 32, 33 für eine Elektrode 18 bzw. ein Elektrodensegment 18c, 18d, 18e vorgesehen sein.

Die Montage des HF-chirurgischen Instrument 1 kann wie nachfolgend beschrieben ablaufen.

Zunächst wird das HF-chirurgische Instrument 1 mit den zwei zueinander in eine Schließstellung bewegbaren Instrumentenbranchen 10, 12 auf eine an sich bekannte Weise gefertigt und bereitgestellt. Insbesondere werden die Instrumentenbranchen 10, 12 mit den Elektroden 18, 20 bestückt.

Die Bestückung der einen bzw. unteren Instrumentenbranche 18 bzw. des Elektrodenträgers kann im Spritzgießverfahren erstellt werden, indem die Elektroden bzw. Elektrodensegmente 18 in das Spritzgießwerkzeug gelegt werden und mit Kunststoff umspritzt werden.

In einem (zeitlich) davon unabhängigen Arbeitsschritt wird die Überbrückungseinrichtung 36, bei der es sich in diesem Ausführungsbeispiel um eine maschinell bestückte Leiterplatte 34 handelt, gefertigt und bereitgestellt. Diese separat ausgebildete Überbrückungseinrichtung 36 wird mittels der Kontaktelemente 26, 28, 30 mit der elektrischen Leitung 22 verbunden, indem die Strom leitenden Adern in die Kontaktelemente 26, 28, 30 eingelegt und diese anschließend die Adern schneidend und klemmend betätigt werden.

Um einen elektrisch leitfähigen (Druck-)Kontakt zwischen dem Federkontaktelementen 32, 33 und der Elektrode 18 herzustellen, wird die Überbrückungseinrichtung 36 derart ausgerichtet in die Aufnahmeöffnung 38 eingeschoben, dass die Federkontaktelemente 32, 33 mit dem Federteil gegen die untere Oberflächenseite 18b der Elektrode 18 gedrückt werden und somit mit dieser in Anlage gebracht werden. Dabei stützt sich die Leiterplatte 34 mit einer Seite, die der die Kontaktelemente 26, 28, 30 und Federkontaktelemente 32, 33 tragenden Seite gegenüberliegt, innerhalb der Aufnahmeöffnung 38 an der Gehäusewand 40 ab. Die Leiterplatte 36 wird so eingebracht, dass sie in axialer Richtung formschlüssig in der Instrumentenbranche 10 aufgenommen und gesichert ist. Durch diese abstützende Anordnung der Leiterplatte 34 wird diese zum einen kraftschlüssig und zum anderen formschlüssig in der Aufnahmeöffnung 38 und in elektrisch leitfähigen Kontakt mit der unteren Oberflächenseite 18b der Elektrode 18 gehalten.

Anstatt die Überbrückungseinrichtung 36 axial in die Instrumentenbranche einzuschieben, kann diese alternativ von der Elektrodenunterseite in die unten offene Instrumentenbranche 10 bzw. den Elektrodenträger eingesetzt werden, so dass die Federkontaktelemente 32, 33 mit dem Federteil gegen die untere Oberflächenseite 18b der Elektrode 18 gedrückt werden und somit mit dieser in Anlage gebracht werden. Anschließend wird die Überbrückungseinrichtung 36 am Elektrodenträger befestigt, z.B. mittels Stifte oder Schrauben. Die Unterseite der Überbrückungseinrichtung 36 bzw. Leiterplatte ist so gestaltet, dass diese die Instrumentenbranche 10 bündig verschließt.

Ausgehend von dem dargestellten Ausführungsbeispiel kann die Erfindung in vielerlei Hinsicht abgewandelt werden. Beispielsweise ist es denkbar, dass die elektrische Leitung 22 beziehungsweise deren einzelne Adern, anstatt mittels der als Schneid- und/oder Klemmelemente ausgeführten Kontaktelementen 26, 28, 30, mittels einer oder mehrerer Lötösen (nicht gezeigt), beispielsweise durch Anlöten, an der Überbrückungseinrichtung 36 beziehungsweise an der Leiterplatte 34 elektrisch leitfähig befestigt werden.

## Patentansprüche

1. HF-chirurgisches Instrument (1) mit zwei zueinander in eine Schließstellung bewegbaren Instrumentenbranchen (10, 12), die jeweils mit zumindest einer Elektrode (18, 20) bestückt sind und mit elektrischer Energie von einer Energiequelle (24) versorgt werden können, und
eine separat ausgebildete Überbrückungseinrichtung (36), welche als eine, vorzugsweise maschinell bestückbare, Leiterplatte (34) ausgebildet ist, die über zumindest einen elektrischen Anschluss oder zumindest eine elektrische Leitung (22) mit der Energiequelle (24) verbindbar ist und zur Versorgung der zumindest einen Elektrode (18, 20) mit elektrischer Energie zumindest eine Kontaktstelle (32, 33) aufweist, in zumindest eine der beiden Instrumentenbranchen (10, 12) derart lösbar einbringbar ist, dass die zumindest eine Kontaktstelle die zumindest eine Elektrode (18, 20) elektrisch kontaktiert, **dadurch gekennzeichnet, dass**
die Überbrückungseinrichtung (36) über eine axiale Öffnung (38) an der proximalen Seite der Instrumentenbranche (10, 12) in axialer Richtung in die Instrumentenbranche (10, 12) einschiebbar ist.

2. HF-chirurgisches Instrument nach Anspruch 1, **dadurch gekennzeichnet, dass** die zumindest eine Kontaktstelle (32, 33) der Überbrückungseinrichtung (36) und/oder die Elektrode (18, 20) ein elektrisch leitfähiges Federkontaktelement (32) aufweist, das einen elektrisch leitfähigen Druckkontakt zwischen der Kontaktstelle und der Elektrode (18, 20) bewirkt.

3. HF-chirurgisches Instrument nach Anspruch 2, **dadurch gekennzeichnet, dass** die in die Instrumentenbranche (10, 20), insbesondere lose, eingebrachte Überbrückungseinrichtung (36) über den Druckkontakt zwischen dem zumindest einen Federkontaktelement (32) und der zumindest einen Elektrode (18, 20) kraftschlüssig in der Instrumentenbranche (10, 12) fixiert ist.

4. HF-chirurgisches Instrument nach Anspruch 2 oder 3, **dadurch gekennzeichnet, dass** das zumindest eine Federkontaktelement (32) in einer vorgesehenen Kontaktposition einen lösbaren Rasteingriff zwischen Instrumentenbranche (10, 12) und Überbrückungseinrichtung (36) herstellt.

5. HF-chirurgisches Instrument nach einem der vorangehenden Ansprüche, **dadurch gekennzeichnet, dass** der zumindest eine elektrische Anschluss der Überbrückungseinrichtung ein Klemm-Schneid-Element (26, 28, 30) zur klemmenden elektrischen Verbindung mit einer mit der Energiequelle (24) verbundenen elektrischen Leitung (22) aufweist.

6. HF-chirurgisches Instrument nach einem der Ansprüche 1 bis 5, **dadurch gekennzeichnet, dass** der zumindest eine elektrische Anschluss durch eine Lötöse zur stoffschlüssigen, elektrischen Verbindung mit einer mit der Energiequelle verbundenen elektrischen Leitung (22) ausgebildet ist.

7. HF-chirurgisches Instrument nach einem der vorangehenden Ansprüche, **dadurch gekennzeichnet, dass** die eine der beiden Instrumentenbranchen (10) mit der Energiequelle (24) direkt verbunden ist und die andere (12) der beiden Instrumentenbranchen (10, 12) mittelbar über die eine Instrumentenbranche (10) mit elektrischer Energie versorgt wird, wobei an der Überbrückungseinrichtung (36) der einen Instrumentenbranche (10) ein erster Kontakt (42) vorgesehen ist, der zumindest in der Schließstellung der beiden Instrumentenbranchen (10, 12) einen entsprechenden zweiten Kontakt an der anderen Instrumentenbranche (12) elektrisch kontaktiert.

8. Verfahren zum Montieren eines HF-chirurgischen Instruments (1) mit zwei zueinander in eine Schließstellung bewegbare Instrumentenbranchen (10, 12), die jeweils mit zumindest einer Elektrode (18, 20) bestückbar sind und mit elektrischer Energie von einer Energiequelle (24) versorgt werden können,
umfassend die Schritte:
Bestücken der Instrumentenbranchen (10, 12) mit jeweils zumindest einer Elektrode (18, 20);
Verbinden einer separat ausgebildeten Überbrückungseinrichtung (36), welche als eine, vorzugsweise maschinell bestückbare, Leiterplatte (34) ausgebildet ist, über zumindest einen Anschluss oder zumindest eine Leitung (22) mit der Energiequelle (24);
und **gekennzeichnet durch** die Schritte:
Lösbares einsetzen der Überbrückungseinrichtung (36), die zur Versorgung der zumindest einen Elektrode (18, 20) mit elektrischer Energie zumindest eine Kontaktstelle aufweist, in zumindest eine Instrumentenbranche (10, 12);
In Anlage bringen der zumindest einen Kontaktstelle der Überbrückungseinrichtung mit der zumindest einen Elektrode (18, 20); und
Einschieben der Überbrückungseinrichtung (36) über eine axiale Öffnung (38) an der proximalen Seite der Instrumentenbranche (10, 12) in axialer Richtung in die Instrumentenbranche (10, 12).

## Claims

1. A HF surgical instrument (1) comprising two instrument branches (10, 12) movable toward each other into a closing position each of which is equipped with at least one electrode (18, 20) and can be supplied with electric power from a power source (24), and
a separately configured bridging means (36), formed as a printed board (34) preferably adapted to be machine-equipped, which is connectable to the power source (24) via at least one electric connection or at least one electric line (22) and includes at least one contact point (32, 33) for supplying the at least one electrode (18, 20) with electric power, can be releasably introduced to at least one of the two instrument branches (10, 12) such that the at least one contact point electrically contacts the at least one electrode (18, 20) **characterized in that**
the bridging means (36) is insertable into the instrument branch (10, 12) in the axial direction via an axial aperture (38) on the proximal side of the instrument branch (10, 12).

2. The HF surgical instrument according to claim 1, **characterized in that** the at least one contact point (32, 33) of the bridging means (36) and/or the electrode (18, 20) have an electrically conductive spring contact element (32) which causes an electrically conductive pressure contact between the contact point and the electrode (18, 20).

3. The HF surgical instrument according to claim 2, **characterized in that** the bridging means (36) introduced, especially loosely, into the instrument branch (10, 20) is non-positively fixed in the instrument branch (10, 12) via the pressure contact between the at least one spring contact element (32) and the at least one electrode (18, 20).

4. The HF surgical instrument according to claim 2 or 3, **characterized in that** the at least one spring contact element (32) produces a releasable locking engagement between the instrument branch (10, 12) and the bridging means (36) in a provided contact position.

5. The HF surgical instrument according to any one of the preceding claims, **characterized in that** the at least one electric connection of the bridging means comprises a clamping-cutting element (26, 28, 30) for a clamping electric connection to an electric line (22) connected to the power source (24).

6. The HF surgical instrument according to any one of claims 1 to 5, **characterized in that** the at least one electric connection is formed by a soldering eyelet for cohesive electric connection to an electric line (22) connected to the power source.

7. The HF surgical instrument according to any one of the preceding claims, **characterized in that** the one of the two instrument branches (10) is directly connected to the power source (24) and the other (12) of the two instrument branches (10, 12) is supplied with electric power indirectly via the one instrument branch (10), wherein at the bridging means (36) of the one instrument branch (10) a first contact (42) is provided which electrically contacts a corresponding second contact at the other instrument branch (12) at least in the closing position of the two instrument branches (10, 12).

8. A method of assembling an HF surgical instrument (1) comprising two instrument branches (10, 12) movable toward each other into a closing position each of which can be equipped with at least one electrode (18, 20) and can be supplied with electric power from a power source (24),
comprising the steps of:
equipping the instrument branches (10, 12) with at least one respective electrode (18, 20);
connecting separately configured bridging means (36), formed as a printed board (34) preferably adapted to be machine-equipped, to the power source (24) via at least one connection or at least one line (22);
and **characterized by** the steps of:
detachably inserting the bridging means (36), which includes at least one contact point for supplying the at least one electrode (18, 20) with electric power, into at least one instrument branch (10, 12);
contacting the at least one contact point of the bridging means with the at least one electrode (18, 20); and
inserting the bridging means (36) into the instrument branch (10, 12) in the axial direction via an axial aperture (38) on the proximal side of the instrument branch (10, 12).

## Revendications

1. Instrument chirurgical HF (1) avec deux branches d'instrument (10, 12) déplaçables l'une par rapport à l'autre dans une position de fermeture, qui sont respectivement équipées d'au moins une électrode (18, 20) et peuvent être alimentées en énergie électrique par une source d'énergie (24), et
un équipement de pontage (36) conçu séparément, lequel est conçu en tant que circuit imprimé (34), qui peut être équipé mécaniquement de préférence, qui peut être relié à la source d'énergie (24) via au moins un raccord électrique ou au moins une conduite électrique (22) et présente au moins un point de contact (32, 33) pour l'alimentation en énergie électrique de la au moins une électrode (18, 20), qui peut être amené de façon amovible dans au moins une des deux branches d'instrument (10, 12) de sorte que le au moins un point de contact (32, 33) entre en contact avec la au moins une électrode (18, 20), **caractérisé en ce que**
l'équipement de pontage (36) peut être poussé dans la direction axiale jusque dans la branche d'instrument (10, 12) via une ouverture (38) axiale sur le côté proximal de la branche d'instrument (10, 12).

2. Instrument chirurgical HF selon la revendication 1, **caractérisé en ce que** le au moins un point de contact (32, 33) de l'équipement de pontage (36) et/ou l'électrode (18, 20) présente un élément de contact à ressort (32) électriquement conducteur qui entraîne un contact de pression électriquement conducteur entre le point de contact et l'électrode (18, 20).

3. Instrument chirurgical HF selon la revendication 2, **caractérisé en ce que** l'équipement de pontage (36), en particulier lâche, amené dans la branche d'instrument (10, 20) est fixé par liaison de force dans la branche d'instrument (10, 12) via la pression de contact entre le au moins un élément de contact à ressort (32) et la au moins une électrode (18, 20).

4. Instrument chirurgical HF selon la revendication 2 ou 3, **caractérisé en ce que** le au moins un élément de contact à ressort (32) établit, dans une position de contact prévue, un encliquetage amovible entre branche d'instrument (10, 12) et équipement de pontage (36).

5. Instrument chirurgical HF selon l'une des revendications précédentes, **caractérisé en ce que** le au moins un raccord électrique de l'équipement de pontage présente un élément de serrage-entaillage (26, 28, 30) pour une liaison électrique de serrage avec une conduite électrique (22) reliée à la source d'énergie (24).

6. Instrument chirurgical HF selon l'une des revendications 1 à 5, **caractérisé en ce que** le au moins un raccord électrique est conçu par un œillet de brasage pour une liaison électrique par liaison de matière avec une conduite électrique (22) reliée à la source d'énergie.

7. Instrument chirurgical HF selon l'une des revendications précédentes, **caractérisé en ce que** la une des deux branches d'instrument (10) est directement reliée à la source d'énergie (24) et l'autre (12) des deux branches d'instrument (10, 12) est indirectement alimentée en énergie électrique via la une branche d'instrument (10), dans lequel, au niveau de l'équipement de pontage (36) de la une branche d'instrument (10), est prévu un premier contact (42) qui, au moins dans la position de fermeture des deux branches d'instrument (10, 12), entre électriquement en contact au niveau de l'autre branche d'instrument (12) avec un second contact correspondant.

8. Procédé de montage d'un instrument chirurgical HF (1) avec deux branches d'instrument (10, 12) déplaçables l'une par rapport à l'autre dans une position de fermeture, qui sont respectivement équipées d'au moins une électrode (18, 20) et peuvent être alimentées en énergie électrique par une source d'énergie (24), qui comprend les étapes consistant à :
équiper les branches d'instrument (10, 12) avec respectivement au moins une électrode (18, 20) ;
relier un équipement de pontage (36) conçu séparément, lequel est conçu en tant que circuit imprimé (34), qui peut être équipé mécaniquement de préférence, à la source d'énergie (24) via au moins un raccord ou au moins une conduite électrique (22) ;
**caractérisé par** les étapes consistant à :
introduire de façon amovible l'équipement de pontage (36), qui présente au moins un point de contact pour l'alimentation en énergie électrique de la au moins une électrode (18, 20), dans au moins une branche d'instrument (10, 12) ;
placer le au moins un point de contact de l'équipement de pontage avec la au moins une électrode (18, 20) ; et
pousser l'équipement de pontage (36) dans la direction axiale jusque dans la branche d'instrument (10, 12) via une ouverture (38) axiale sur le côté proximal de la branche d'instrument (10, 12).
